**Europäisches Patentamt**

**European Patent Office**

**Office européen des brevets**

(19)

(11) Veröffentlichungsnummer: **0 053 999**

**B1**

(12) **EUROPÄISCHE PATENTSCHRIFT**

(45) Veröffentlichungstag der Patentschrift:
18.03.87

(51) Int. Cl.⁴: **A 61 B 17/58**

(21) Anmeldenummer: **81810467.1**

(22) Anmeldetag: **20.11.81**

(54) Osteosynthetische Vorrichtung und dazu passende Bohrlehre.

(30) Priorität: **20.11.80 CH 8599/80**

(43) Veröffentlichungstag der Anmeldung:
**16.06.82 Patentblatt 82/24**

(45) Bekanntmachung des Hinweises auf die Patenterteilung:
**18.03.87 Patentblatt 87/12**

(84) Benannte Vertragsstaaten:
**BE CH DE FR LI SE**

(56) Entgegenhaltungen:
**CH-A-566 767**
**DE-A-2 340 880**
**FR-A-1 505 513**
**FR-A-2 480 106**
**US-A-3 779 240**

(73) Patentinhaber: **Synthes AG Chur, Grabenstrasse 15,
CH- 7002 Chur (CH)**

(72) Erfinder: **Klaue, Kaj, Hôpital d'arrondissement
Sierre, CH- 3960 Sierre (CH)**

(74) Vertreter: **Zumstein, Fritz, Dr., Dr. F. Zumstein
sen Dr. E. Assman Dipl.- Ing. F. Klingseisen Dr.
F. Zumstein jun. Bräuhausstrasse 4, D-8000
München 2 (DE)**

## Beschreibung

Die Erfindung betrifft eine Vorrichtung zur Stabilisierung des Bereichs eines Knochenbruches oder einer Osteotomie nach dem Oberbegriff des Anspruchs 1.

Aus der CH-A-462 375 ist eine osteosynthetische Druckplatte gemäß dem Oberbegriff des Anspruchs 1 bekannt, bei der mindestens ein Schraubenloch als sich in der Plattenlängsrichtung erstreckender Schiebeschlitz ausgebildet ist, wobei die den Schraubensitz bildende Ansenkung auf der Außenseite der Platte an einem Schlitzende eine Anschrägung aufweist, so daß beim Einsenken des Schraubenkopfes an diesem Ende des Schiebeschlitzes die Platte zwangsweise in der Längsrichtung von der Frakturstelle wegverschoben wird. Am anderen Schlitzende ist die Ansenkung als dem Schraubenkopf angepaßte, die Längsverschiebung der Platte begrenzende Endanschlagfläche ausgebildet. Die für diese Druckplatte vorgesehenen Kugelkopfschrauben werden senkrecht zur Plattenebene eingebracht.

Der Stand der Technik der Kompressions-Osteosynthese, sowohl vorrichtungsmässig als auch operationstechnisch, ist zusammenfassend in der folgenden Veröffentlichung beschrieben:

a) M. E. Müller, M. Allgöwer, R. Schneider und H. Willenegger, Manual der Osteosynthese (AO-Technik), 2. Auflage, Springer-Verlag Berlin Heidelberg New York 1977

b) B. Claudi u.a., Hel. chir. Acta 46 (1979), 177-182.

Weiter ist bezüglich Vorrichtungen auf die folgenden Veröffentlichungen zu verweisen:

c) CH-PS 462 373
d) CH-PS 468 824
e) CH-PS 600 862
f) CH-PS 611 147
g) CH-PS 613 616
h) CH-PS 613 858

Bis jetzt wurde bei der Kompressions-Osteosynthese die Kompressionsplatte überwiegend nach dem Zuggurtungsprinzip angewendet. Dabei wird die Platte einseitig auf den zu stabilisierenden Bereich an die auf Zug beanspruchte Seite des Knochens angebracht und nach Vorspannung mittels Schrauben lotrecht zur Knochen- und Plattenlängsachse mit dem Knochen verschraubt; die der Platte gegenüberliegende Muskulatur sowie die physiologische Belastung schliesst durch zusätzliche dynamische Kompression den Frakturspalt (vgl. Veröffentlichung a)).

Statische, interfragmentäre Kompression kann durch einfache Zugschrauben ohne Verwendung einer Platte erreicht werden (Veröffentlichung a)).

Neuerdings wurde auch bereits vorgeschlagen, die Stabilisierung mittels Platte durch Hinzufügen einer schiefwinklig zur Knochen- und Plattenlängsachse verlaufenden, die Frakturspaltebene durchdringenden schrägen Zugschraube, deren Axialkraft eine zusätzliche interfragmentäre Kompression bewirkt, zu verbessern (vgl. Veröffentlichung b), Seite 178 und Abb. 2B).

Der Anwendung der letzterwähnten Methode waren bisher jedoch enge Grenzen gesetzt, da die Geometrie der bisher bekannten Kompressionsplatten und Schrauben nur eine ungenügende Schwenkung der Schrauben gegenüber der Querebene und in geschwenktem und voll eingeschraubtem Zustande praktisch keine Längsbewegung der Schrauben, welche über ihre ganze Länge mit Gewinde versehen waren, in den Widerlagern der Löcher der Kompressionsplatte zuliess.

Der Erfindung liegt die Aufgabe zugrunde, eine Vorrichtung der eingangs angegebenen Art so auszubilden, daß Zugschrauben schräg eingesetzt und in dieser Stellung auch verschoben werden können, wobei eine ausreichende Schwenkung der Schrauben möglich sein soll.

Diese Aufgabe wird durch die Merkmale im Kennzeichen des Anspruchs 1 gelöst. Durch die Abschrägung auf der Innenseite der Platte an den stirnseitigen Rändern der Löcher können die Schrauben um bis zu 45° gegenüber einer Querebene zur Lochlängsachse verschwenkt werden, wobei sie in der Lochlängsrichtung in geschwenktem Zustand verschiebbar sind. Dabei können die schräg eingesetzten Zugschrauben als ausschließliches oder zusätzliches Kompressionselement dienen. Diese Ausgestaltung gestattet eine kurze Auslegung der Langlöcher bei maximaler Schwenkbarkeit der Schraube in Verbindung mit deren horizontaler Verschiebbarkeit. Es wird ein Kippmoment an der schräg eingesetzten Schraube erzeugt, das zu einer Kompression an den beiden einander gegenüberliegenden Frakturstellen eines Röhrenknochens führt.

Vorteilhafte Ausgestaltungen der Erfindung sind in den weiteren Ansprüchen angegeben.

Beispielsweise Ausführungsformen der Erfindung sowie der Stand der Technik werden nachstehend anhand der Zeichnung näher erläutert. Es stellen dar:

Fig. 0 einen Längsschnitt durch eine mittels einer dynamischen Kompressionsplatte gemäss dem Stand der Technik fixierten Fraktur;

Fig. 1 eine schematische Darstellung der bei Anwendung des Prinzips der schrägen Kompressionsschraube mit der erfindungsgemässen Vorrichtung entstehenden Kräfte und Momente;

Fig. 2 einen Längsschnitt durch eine Ausführungsform der erfindungsgemässen Vorrichtung in appliziertem Zustand an einer mehrfragmentären Fraktur;

Fig. 3 einen Querschnitt durch eine Ausführungsform der erfindungsgemässen Vorrichtung, bei welcher der Rand des Loches der vom Knochen abgewandten Seite der Platte gegenüber dem das Widerlager bildenden Teil der Wandungen nach aussen abgeschrägt ist;

Fig. 4 einen Längsschnitt durch eine Ausführungsform der erfindungsgemässen Vorrichtung während der Herstellung durch Fräsen eines Loches mittels eines Kugelfräsers;

Fig. 5 einen Längsschnitt durch eine Ausführungsform der erfindungsgemässen Vorrichtung während des Ausfräsens einer trichterförmigen Erweiterung;

Fig. 6 einen Längsschnitt durch eine erste Ausführungsform einer Bohrlehre;

Fig. 7 einen Längsschnitt durch eine erfindungsgemässe Vorrichtung mit der in Fig. 6 dargestellten Bohrlehre in Applikationslage;

Fig. 8 und 9 eine zweite Ausführungsform einer Bohrlehre in Längs- bzw. Querschnitt; und

Fig. 10 die zeichnerische Darstellung der Zusammenhänge zwischen den Abmessungen der Bohrlehre, der Löcher und der Schrauben.

Die Bohrlehre ist Gegenstand der Teilanmeldung 0 173 267.

In Fig. 0 ist ein Längsschnitt durch eine mittels einer dynamischen Kompressionsplatte fixierte Fraktur dargestellt. Diese Darstellung entspricht dem Stand der Technik. Die Kompressionsplatte 1 liegt mindestens angenähert bündig auf dem Knochen 5 auf, weist mindestens zwei Löcher 3 auf (nur eins dargestellt) und ist dort mittels einer Kugelkopfschraube 2 am Knochen 5 fixiert. Letztere stösst am Lochrand 4 an, was beim Einschrauben zum Abdrücken des Kopfes der Kugelkopfschraube 2, d.h. Verschiebung der Schraube in Richtung der Platten- und Knochenlängsachse und des darunterliegenden Knochens, in Frakturrichtung führt. Das Fixieren der Kompressionsplatte 1 bewirkt die Entstehung von entgegengesetzt gerichteten Kräfte A und B an der Bruchstelle 6, 7. Dabei ist die Kraft B, welche die Bruchstelle 7 an der der Kompressionsplatte 1 abgewandten Seite auseinanderzieht, unerwünscht. Es wurde schon versucht, durch verschiedene Massnahmen der Kraft B entgegenzuwirken oder sie zu eliminieren, beispielsweise durch Biegen der Kompressionsplatte i (Literatur b)).

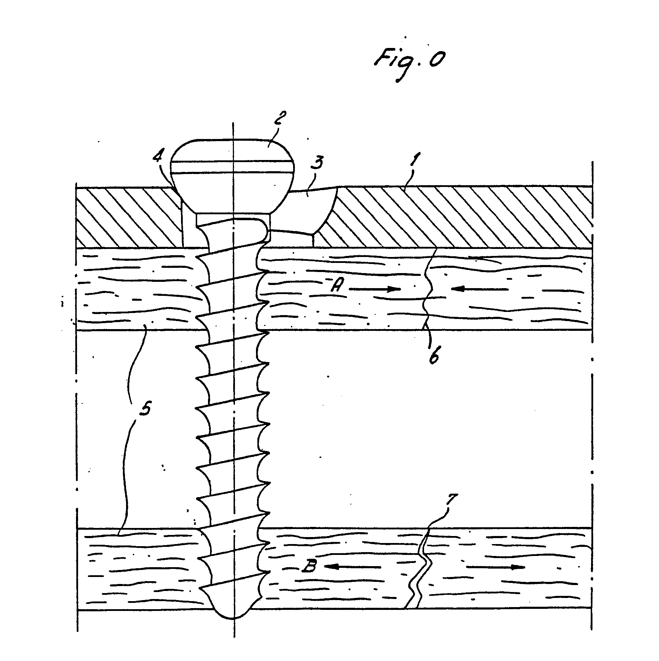

Fig. 1 zeigt dagegen die ebenfalls entgegengesetzt gerichteten Kräfte C, D, die bei Anwendung der erfindungsgemässen Vorrichtung entstehen. Die mit mehr als einem Loch 10 (nur eins dargestellt) versehene Platte 12 liegt wieder mindestens angenähert bündig auf dem Knochen 14 auf. Die Schraube 16 weist zwischen Schraubenkopf 17 und Gewindeteil 19 einen gewindefreien Teil 18 auf, dessen Durchmesser d mindestens ebenso gross ist wie der Außendurchmesser des Gewindeteils 19, und ist schräg in den Knochen 14 eingesetzt, und zwar derart, dass sie mit einer lotrecht auf die Lochlängsachse stehenden Ebene E einen Neigungswinkel β von 45° bildet. Die Schraube 16 ist in zwei Stellungen 21 und 22 dargestellt, welchletztere sie durch Gleiten in der Gleitbahn 24 einnehmen kann. Durch das Gleiten der Schraube 16 entsteht eine Hebelwirkung, durch welche die Schraubenachse um den Winkel γ

gekippt wird. Die Hebelwirkung ist derart, wie wenn die Kräfte in den Punkten 26, 28 angreifen würden und 27 der Stützpunkt des Hebels wäre. Auf die Bruchstelle 30 des Knochens 14 wirken die Kräfte C, D, welche im Gegensatz zur Fig. 0 die Bruchstücke an allen Stellen zusammendrücken.

In Fig. 2 wird ein Längsschnitt durch eine Ausführungsform der erfindungsgemässen Vorrichtung in appliziertem Zustand an einer mehrfragmentären Fraktur 30 dargestellt. Die mit mehreren Löchern 10 versehene Platte 12 liegt wieder mindestens angenähert bündig auf dem Knochen 14 auf, welcher mehrere Frakturen 30 aufweist.

Die drei Schrauben 32, 33, 34 weisen zwischen Schraubenkopf 17 und Gewinde 19 einen gewindefreien Teil 18 auf. Die Schrauben 32, 33, 34 sind schräg eingesetzt, und zwar derart, dass sie mit einer lotrecht auf die Lochlängsachse stehenden Ebene E einen Neigungswinkel β bilden, welcher für die Schrauben 32, 33, 34 verschieden gross und vom Verlauf der Bruchstelle 30 abhängig ist. Die Schrauben 32, 33, 34 können in der jeweiligen Gleitbahn 24 gleiten. Bei den Schrauben 36, 37 handelt es sich um Plattenfixationsschrauben, deren Achsen lotrecht zur Lochlängsachse stehen. Sie dienen nur zur Befestigung der Platte 12 am Knochen 14. Währenddem die Anzahl der schräg eingesetzten Schrauben 32, 33, 34 von Art und Anzahl der Bruchstellen abhängig ist, werden vorzugsweise mindestens zwei Plattenfixationsschrauben, vorzugsweise an den beiden Längsenden der Platte 12, eingesetzt.

Die Schrauben können entweder mit einem solchen Gewinde versehen sein, welches das Vorschneiden der Gewinde im Bohrloch erforderlich macht oder auch selbstschneidende Gewinde aufweisen, welche in des gewindefreie Bohrloch eingeschraubt werden. Es können auch Schrauben verwendet werden, die über die ganze Länge des Schaftes mit Gewinde versehen sind.

Fig. 3 ist ein Querschnitt durch eine Ausführungsform der erfindungsgemässen Vorrichtung, bei welcher der Rand des Loches 10 auf der vom Knochen abgewandten Seite der Platte 12 gegenüber dem das Widerlager 23 bildenden Teil der Wandungen durch eine Abschrägung 40 nach außen versetzt ist.

Fig. 4 zeigt einen Längsschnitt durch eine Ausführungsform der erfindungsgemässen Vorrichtung während der Herstellung durch Fräsen eines Loches 10 mit einem Kugelfräser 42. Der Mittelpunkt 44 des Kopfes des Kugelfräsers 42 bewegt sich während des Fräsvorganges entlang der Strecke X. Vorteilhaft werden diese Löcher durch computergesteuerte Kugelfräser hergestellt.

In Fig. 5 ist ein Längsschnitt durch eine Ausführungsform der erfindungsgemässen Vorrichtung dargestellt. Es handelt sich um das Ausfräsen der trichterförmigen Erweiterung 46 des Loches 10 auf der dem Knochen zugewandten Seite der Platte 12.

Die Länge des Loches ist in Verbindung mit dem Schraubenschaft so ausgelegt, daß die Schraube unter einem Neigungswinkel $\beta$ von bis zu 45° gegenüber der lotrecht auf der Lochlängsachse L stehenden Ebene E um mindestens ein Drittel des Schaftdurchmessers d, gemessen lotrecht zur Schraubenlängsachse S, gleiten kann (Fig.1, 2).

Das durch die längsseitigen Wandungen des Loches 10 gebildete Widerlager 23 kann mindestens teilweise durch den Mantel eines Zylinders definiert sein, dessen Achse zur Lochlängsachse L parallel verläuft (Fig.3, 4). Es ist aber auch möglich, daß das Widerlager 23 durch eine Ebene definiert wird, die sich mit der Abschrägung 40 in Fig. 3 in einer zur Lochlängsachse L parallelen Gerade schneidet.

Die Längsachse L des Loches 10 oder die Längsachsen mindestens eines Teils der Löcher können parallel, schiefwinkelig oder auch rechtwinkelig zur Längsachse der Platte verlaufen

Die Ränder der Löcher auf der am Knochen anliegenden Seiteder Platte 12 werden mindestens auf der frakturnahen Stirnseite des Loches 10 durch eine Abschrägung gegenüber der lichten Lochweite nach außen versetzt, wie dies z.B. Fig. 1 zeigt, so daß die Schraube auch nahe des Lochendes schräg eingesetzt werden kann, ohne mit dem Schaft am Lochrand anzuliegen. Eine solche stirnseitige Abschrägung kann auch auf der vom Knochen abgewandten Seite der Platte 12 an der frakturfernen Stirnseite des Loches vorgesehen werden.

Die längsseitigen Wandungen des Loches 10 sind als Gleitbahn 24 ausgebildet, in welcher der Schraubenkopf sich parallel zur jeweiligen Schraubenlängsachse S verschieben kann, wobei das Loch 10 und die Schraube 16 bzw. 32 bis 37 so ausgebildet, sind, daß die Schraube unter einem Neigungswinkel $\beta$ von bis zu 45° eingebracht werden und im Loch in dieser Stellung gleiten kann.

Die Ränder der Löcher 10 sind mindestens teilweise auf der vom Knochen abgewandten Seite der Platte 12 gegenüber dem das Widerlager 23 bildenden Teil der Wandungen durch eine Abschrägung bzw. eine abgeschrägte Fläche 40 nach außen versetzt. Durch diese Fläche 40 werden die beim Herstellungsvorgang auftretenden Kanten gebrochen, so daß beim Biegen und Verwinden der Platte die dazu benötigten Instrumente die Gleitbahn nicht beschädigen können. In entsprechender Weise sind die Ränder der Löcher 10 auf der am Knochen anliegenden Seite der Platte 12 gegenüber der lichten Lochweite über mindestens einen Teil des Lochumfanges durch eine abgeschrägte Fläche 46 nach außen versetzt.

Die Lochränder sind auf der am Knochen anliegenden Seite der Platte 12 mindestens auf der frakturnahen Stirnseite des Loches 10 und/oder auf der vom Knochen abgewandten Seite der Platte 12 an der frakturfernen Stirnseite des Loches gegenüber des lichten Loches durch eine Abschrägung nach außen versetzt.

Die erfindungsgemässe Vorrichtung weist gegenüber dem Stand der Technik verschiedene Vorteile auf.

Wie in Fig. 0 gezeigt, wird die transkortikale Frakturspalte mit einer Vorrichtung gemäss dem Stand der Technik auseinandergezogen. Durch die schräge Lage der Schraube in der erfindungsgemässen Vorrichtung wird eine doppelte Kompression möglich, da durch die Hebelarmwirkung (Fig. 1) nicht nur wie gemäss dem Stand der Technik die ciskortikale Frakturspalte sondern auch die transkortikale Frakturspalte zusammengedrückt wird. Entsprechend sind bei der Verwendung der erfindungsgemässen Vorrichtung keine zusätzlichen Massnahmen, wie Biegen der Platte, zum Schliessen der transkortikalen Frakturspalte erforderlich.

Beim Anbringen einer Kompressionsplatte gemäss dem Stand der Technik ist der Spannweg, d.h. die Länge der Strecke, entlang welcher die Schraube samt dem von ihr gefassten Knochenteil in Richtung Fraktur geschoben wird, schon im voraus festgelegt. Demgegenüber kann die Kompression mit der erfindungsgemässen Vorrichtung während des Einbringens der Schraube, infolge der Gleitmöglichkeit in der Gleitbahn, den vorherrschenden Umständen angepasst werden, da nicht die ganze mögliche Lochlänge ausgenutzt werden muss.

Der Kopf der Schraube der erfindungsgemässen Vorrichtung wird bei der Verwendung nicht plastisch abgeknickt, d.h. der am Kopf der Schraube anschliessende Teil des Schraubenschaftes wird infolge Kippen der Schraubenachse um den Winkel $\gamma$ nicht in wesentlichem Ausmasse plastisch verbogen.

Bei Fixierung der Frakturen mittels einer Platte sollte letztere möglichst schmal und dünn sein, da unter der Platte eine Auflösung der Kortikalis, sogenannte Spongiosierung, stattfindet. Dieses Phänomen wird auch als Stressprotektion bezeichnet, wobei vermutet wird, dass es infolge Schutz des darunterliegenden Knochenbereiches gegen äussere Beanspruchung zustande kommt. Die Platte der erfindungsgemässen Vorrichtung kann sehr dünn und schmal gestaltet werden, da infolge der transfragmentären Lage der Schraube die mechanische Belastung der Platte geringer wird. Bei Vorrichtungen gemäss dem Stand der Technik entsprach die Toleranz bezüglich der exzentrischen Schraubenlage im Plattenloch der Plattendicke. Da gemäss der Erfindung die Schraube in Richtung der Plattenachse und in geschwenkter Lage gleitbar und schwenkbar ist, kann eine fast beliebig dünne Platte eingesetzt werden, weil die Dickentoleranz der Platte nicht ausgenutzt werden muss.

Obwohl in den Figuren nur Röhrenknochen dargestellt sind, kann die erfindungsgemässe Vorrichtung selbstverständlich auch zur Stabilisierung des Bruchbereiches in anderen Knochen eingesetzt werden.

Fig. 6 zeigt einen Längsschnitt durch eine Bohrlehre, bei welcher das Lager 48 kugelförmig ausgebildet ist. Dieses weist den Radius r auf und ist bezüglich des Kugelmittelpunktes 54 exzentrisch mit der Bohrbüchse 52 verbunden. Letztere weist einen als Halterung dienenden Griff 58 auf.

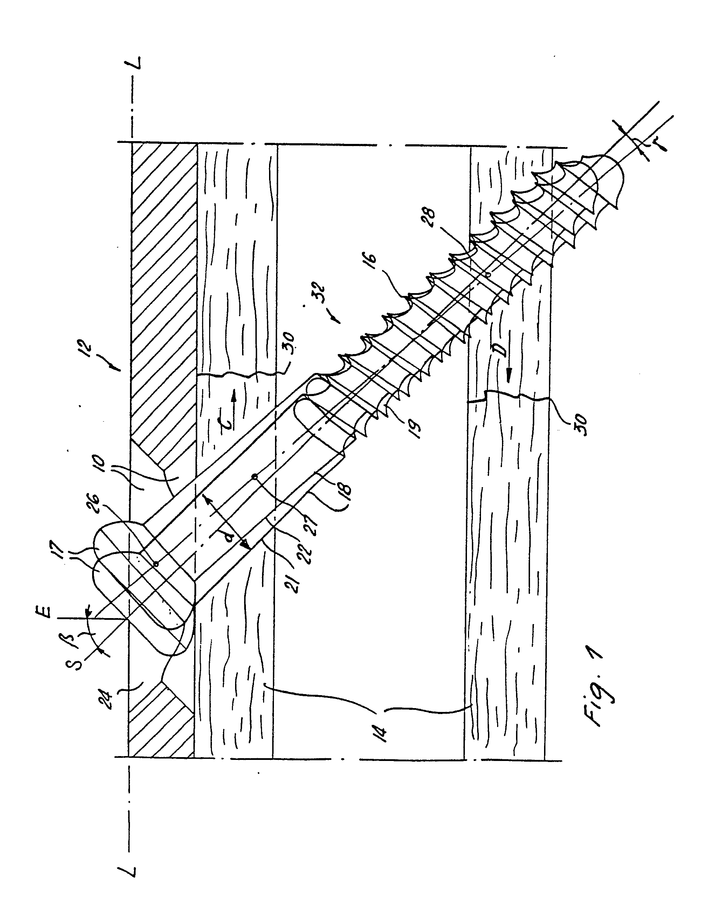

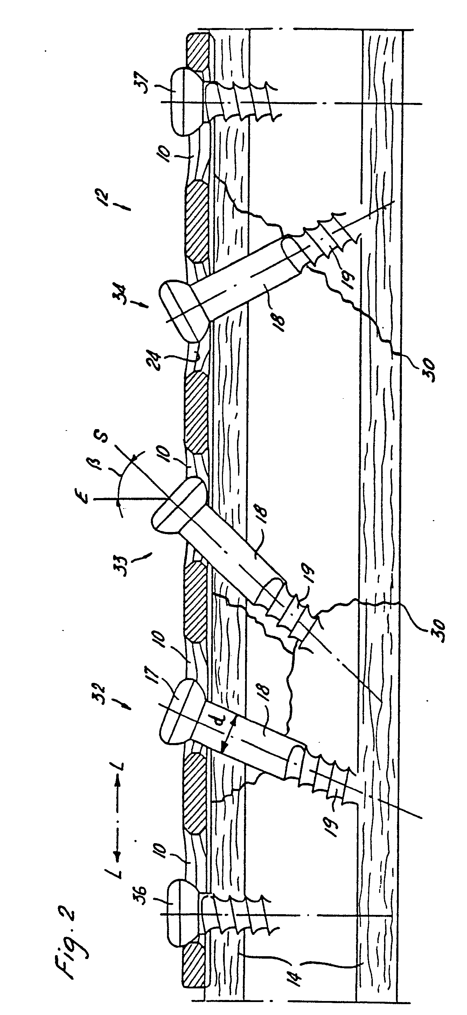

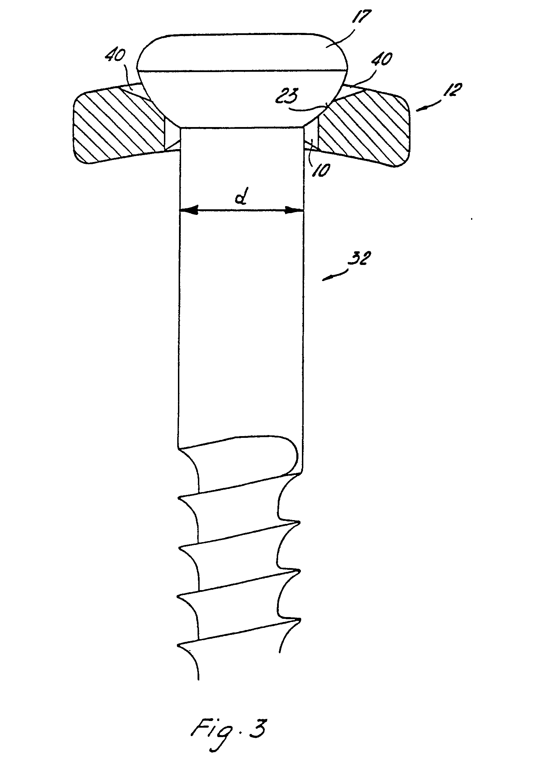

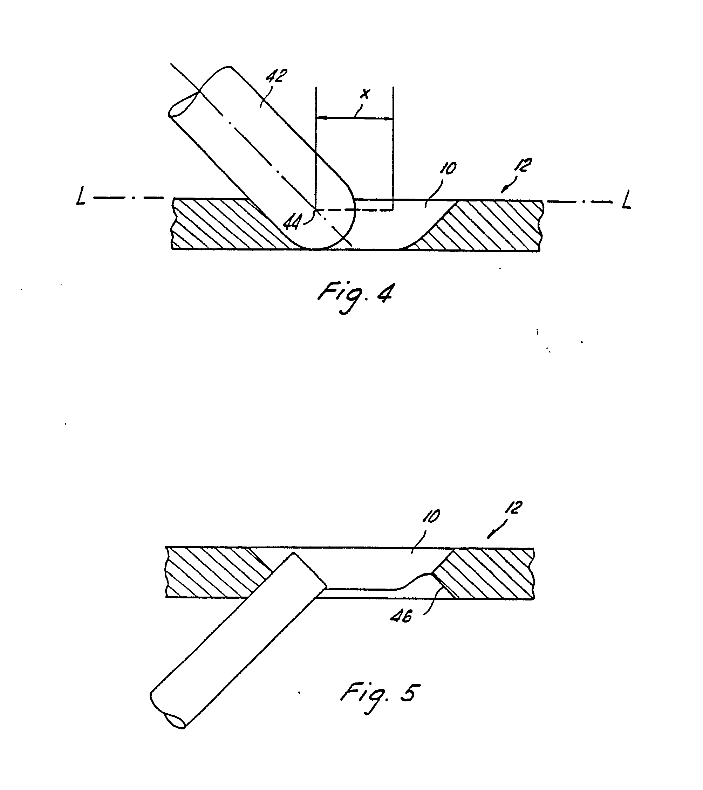

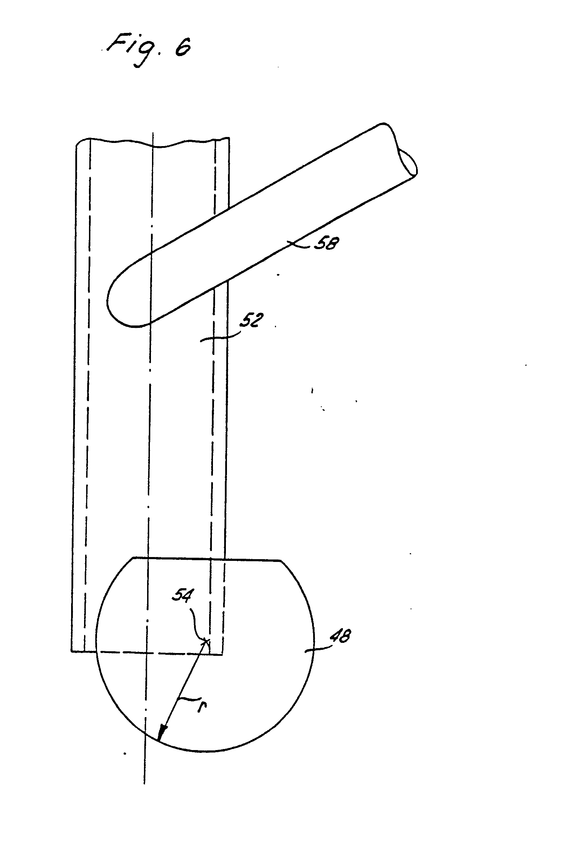

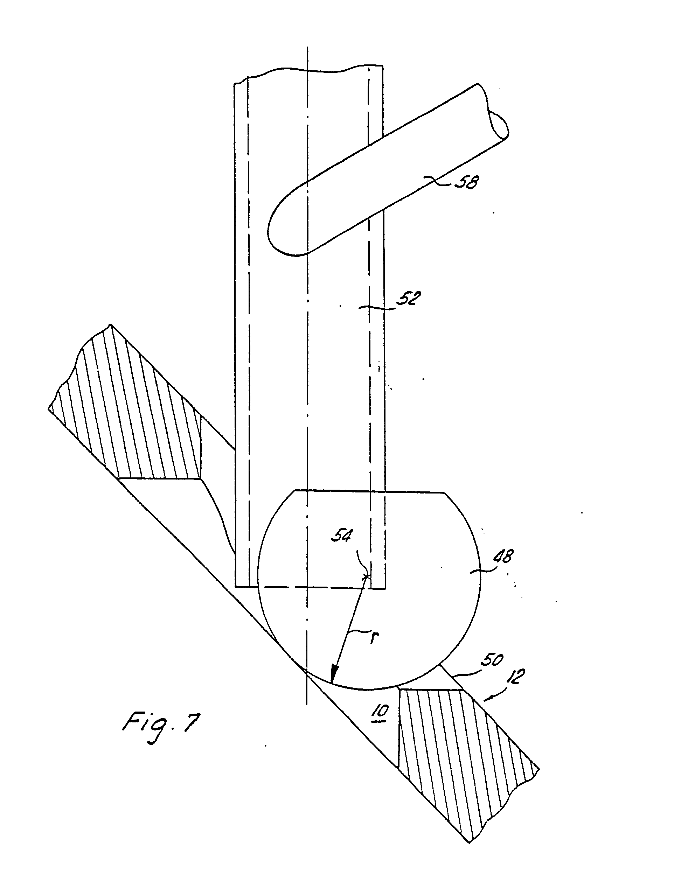

Fig. 7 zeigt einen Längsschnitt durch die Platte 12 der erfindungsgemässen Vorrichtung mit der angelegten Bohrlehre nach Fig. 6. Das kugelförmige Lager 48 mit dem Radius r liegt in der Gleitbahn am frakturnahen Ende 50 des Loches 10. Die zur Führung des Bohrers bestimmte Bohrbüchse 52 ist bezüglich des Kugelmittelpunktes 53 exzentrisch mit dem kugelförmigen Lager 48 verbunden. Der Griff 58 ist an der Bohrbüchse 52 befestigt und dient als Halterung für die Bohrlehre.

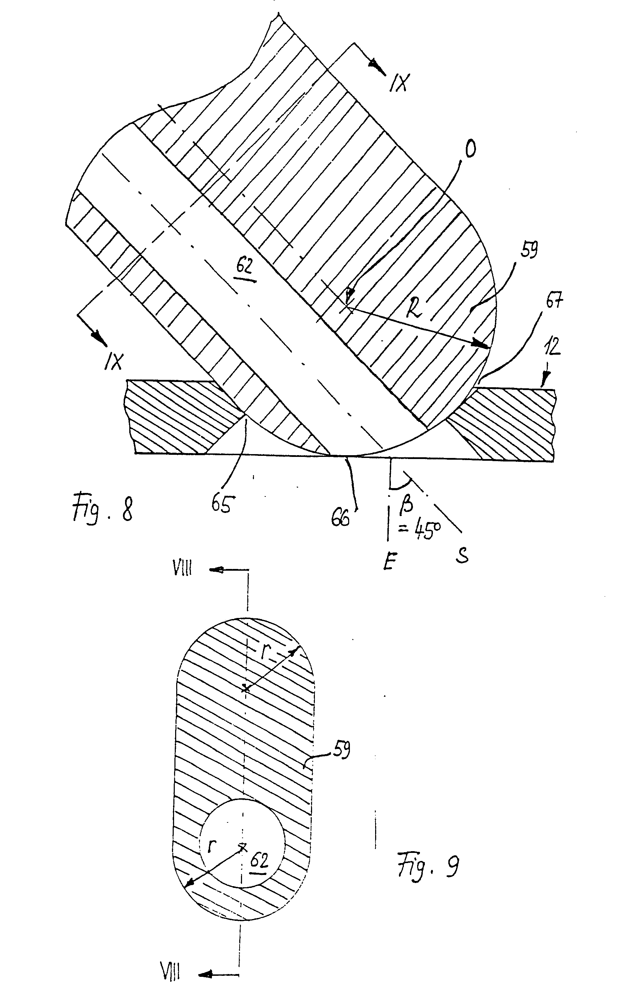

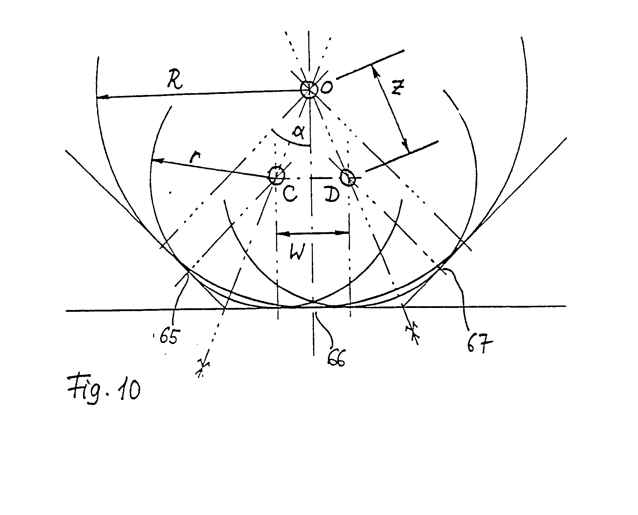

Fig. 8 und 9 zeigen eine zweite Ausführungsform einer Bohrlehre, und zwar Fig.8 einem Längsschnitt entlang der Linie VIII-VIII in Fig. 9 und in Verbindung mit einer erfindungsgemässen Platte 12, Fig. 9 für sich allein einem Querschnitt nach der Linie IX-IX in Fig. 8.

Diese Bohrlehre weist ein kalottenartiges Lager 59 auf, in welches die Bohrbüchse 62 zur Führung des Bohrers exzentrisch eingelassen ist. Der in einer Schnittebene quer zur Lochlängsachse L liegende Radius r des kalottenartigen Lagers 59 (Fig. 9) ist gleich dem Kugelradius der Kugelköpfe der Schrauben, während der in einer die Lochlängsachse L mitumfassenden Schnittebene VIII-VIII liegende Radius n des kalottenartigen Lagers 59 (Fig. 8) grösser als der Kugelradius der Kugelköpfe der Schrauben ist.

Die kongruenten Kontaktstellen zwischen Plattenloch 10 und Bohrlehre sind mit 65, 66 und 67 bezeichnet. Mit zunehmendem Radius R liegt schliesslich das kalottenartige Lager 59 auf der Geleitbahn 24 nicht mehr auf, sondern nur noch an den stirnseitigen Lochenden, so dass die Kontaktstelle 66 verschwindet und nur noch eine Zweipunktlagerung an den Kontaktstellen 65 und 67 stattfindet.

Durch diese Ausgestaltung der Bohrlehre wird zunächst einmal ihre optimale Zentrierung im Plattenloch 10 und in Richtung der Lochlängsachse L erreicht.

Bei einem Neigungswinkel β der Schraube bzw des Bohrers von weniger als 45°, gemessen zwischen E und S, kommt der Schraubenkopf 17 beim Eindrehen der Schraube 16 auf die durch die längsseitigen Wandungen des Plattenloches 10 gebildete Gleitbahn 24, (Fig. 1). Dadurch wird eine besonders wirksame Kompression erreicht.

Bei einem Neigungswinkel β von 45° kommt der Schraubenkopf 17 beim Eindrehen der Schraube 16 direkt auf den Anfang der Gleitbahn 24 zu liegen. Die Kraft, die zwischen Schraube 16 und Platte 12 dabei übertragen wird, ist bei diesem extremen Winkelverhältnis am grössten und dürfte jedenfalls für eine wirksame Kompression genügen.

Bei senkrechter Stellung, d.h. bei einem Neigungswinkel β von 0°, kann die Bohrlehre auch als normale exzentrische Bohrlehre für die zum Stand der Technik gehörende Kompressions-Osteosynthese nach Fig. 0 eingesetzt

Der Fuss der Bohrlehre kann mit Arretierungen versehen sein (in der Zeichnung nicht dargestellt), welche die Festlegung eines bestimmten Neigungswinkels β zwischen 0 und 45° erleichtert und auch eine leichte Seitenschwenkung ermöglichen.

Die der Ausführungsform der Bohrlehre nach Fig. 8 und 9 zugrundeliegenden Zusammenhänge zwischen den beiden Radien r und R des kalottenartigen Lagers, dem parallel zur Lochlängachse gemessenen Gleitweg W der Schraube im Loch und dem Neigungswinkel α der Gleitbahn sind aus Fig. 10 ersichtlich.

Die kongruenten Kontaktstellen sind gleich wie in Fig. 8 mit 65, 66 und 67 bezeichnet. Die Zentren der Schraubenköpfe bei Extremlagen der Schrauben auf der Gleitbahn 24 sind mit C und D bezeichnet. Der Abstand der Punkte C und D entspricht dem horizontalen Gleitweg W.

Dabei gelten die Beziehungen:

$$R = r + \left[ \cos \frac{\alpha}{2} \cdot z \right] \qquad (1)$$

und

$$z = \frac{W}{2} \cdot \frac{1}{\sin \frac{\alpha}{2}} \qquad (2)$$

Daraus ergibt sich der Zusammenhang zwischen den oben erwähnten Grössen:

$$R = r + \left[ \frac{W}{2} \cdot ctg \frac{\alpha}{2} \right] \cdot$$

## Patentansprüche

1. Vorrichtung zur Stabilisierung des Bereiches eines Knochenbruches oder einer Osteotomie bei der Kompressions-Osteosynthese, bestenend aus einer zur Auflage auf den zu stabilisierenden Bereich des Knochens (14) bestimmten Platte (12), die mittels Kugelkopfschrauben (16; 32-37) an den Knochenteilen fixierbar ist, wobei die Platte (12) mindestens ein länglich ausgebildetes Loch (10) aufweist, dessen längsseitige Wandungen ein gegenüber der Plattenoberfläche abgesenktes Widerlager (23) für den Schraubenkopf (17) bilden, das zur Lochlängsachse (L) parallel verläuft, dadurch gekennzeichnet, daß der Rand des Loches (10) auf der am Knochen anliegenden Seite der Platte (12) mindestens auf der frakturnahen Stirnseite des Loches (10) gegenüber der lichten Lochweite durch eine Abschrägung nach außen versetzt ist.

2. Vorrichtung nach Anspruch 1, dadurch gekennzeichnet, daß das durch die längsseitigen Wandungen des Loches (10) gebildete Widerlager (23) mindestens teilweise durch den Mantel eines Zylinders definiert ist, dessen Achse zur Lochlängsachse (L) parallel verläuft.

3. Vorrichtung nach Anspruch 1, dadurch gekennzeichnet, daß das durch die längsseitigen Wandungen des Loches (10) gebildete Widerlager (23) mindestens teilweise durch Ebenen definiert ist, die sich in einer zur Lochlängsachse (L) parallelen Geraden schneiden.

4. Vorrichtung nach Anspruch 1, dadurch gekennzeichnet, daß die Lochlängsachse (L) des Loches (10) oder die Längsachsen mindestens eines Teils der Löcher parallel zur Längsachse der Platte (12) verlaufen.

5. Vorrichtung nach Anspruch 1, dadurch gekennzeichnet, daß die Längsachse (L) des Loches (10) oder die Längsachsen mindestens eines Teils der Löcher schiefwinkelig zur Längsachse der Platte (12) verlaufen.

6. Vorrichtung nach Anspruch 1, dadurch gekennzeichnet, daß die Längsachse (L) des Loches (10) oder die Längsachsen mindestens eines Teils der Löcher rechtwinkelig zur Längsachse der Platte (12) verlaufen.

7. Vorrichtung nach Anspruch 1, dadurch gekennzeichnet, daß der Rand des Loches (10) mindestens teilweise auf der vom Knochen abgewandten Seite der Platte (12) gegenüber dem das Widerlager (23) bildenden Teil der Wandung durch eine abgeschrägte Fläche (40) nach außen versetzt ist.

8. Vorrichtung nach Anspruch 1, dadurch gekennzeichnet, daß sich die längsseitigen Wandungen (10) auf der am Knochen anliegenden Seite der Platte (12) gegenüber der lichten Lochweite durch eine abgeschrägte Fläche (46) nach außen versetzt sind.

9. Vorrichtung nach Anspruch 7 oder 8, dadurch gekennzeichnet, daß der Rand des Loches (10) auf der vom Knochen abgewandten Seite der Platte (12) an der Stirnseite des Loches gegenüber der lichten Lochweite durch eine Abschrägung nach außen versetzt ist.

10. Vorrichtung nach den vorhergehenden Ansprüchen, dadurch gekennzeichnet, daß die Länge des Loches (10) einem Verschiebeweg von mindestens einem Drittel des Schaftdurchmessers (d) der darin eingesetzten Schraube entspricht.

11. Vorrichtung nach Anspruch 1, dadurch gekennzeichnet, daß bei mindestens einem Teil der Schrauben (16; 32-37) der Schraubenschaft zwischen dem Schraubenkopf (17) und dem Gewindeteil (19) ein gewindefreies zylindrisches Zwischenstück (18) aufweist, dessen Durchmesser (d) mindestens gleich groß ist wie der Außendurchmesser des Gewindeteils (19).

**Claims**

1. Device for stabilizing the region of a fracture or of an osteotomy in compression osteosynthesis, consisting of a plate (12) which is intended to bear on the region of the bone (14) to be stabilized and which can be fixed to the bone parts by means of ball-headed screws (16; 32-37), the plate (12) having at least one elongate hole (10), of which the walls on its longitudinal sides form for the screw head (17) an abutment (23) lowered relative to the plate surface and extending parallel to the longitudinal axis (L) of the hole, characterized in that, on the side of the plate (12) adjacent to the bone, the edge of the hole (10) is offset outwards relative to the clear hole width by means of a bevel at least on the end face of the hole (10) located near the fracture.

2. Device according to Claim 1, characterized in that the abutment (23) formed by the walls of the hole (10) which are located on its longitudinal sides is defined at least partially by the outer surface of a cylinder, the axis of which extends parallel to the longitudinal axis (L) of the hole.

3. Device according to Claim 1, characterized in that the abutment (23) formed by the walls of the hole (10) which are located on its longitudinal sides is defined at least partially by two planes which intersect one another in a straight line parallel to the longitudinal axis (L) of the hole.

4. Device according to Claim 1, characterized in that the longitudinal axis (L) of the hole (10) or the longitudinal axes of at least some of the holes extend parallel to the longitudinal axis of the plate (12).

5. Device according to Claim 1, characterized in that the longitudinal axis (L) of the hole (10) or the longitudinal axes of at least some of the holes extend at an oblique angle relative to the longitudinal axis of the plate (12).

6. Device according to Claim 1, characterized in that the longitudinal axis (L) of the hole (10) or the longitudinal axes of at least some of the holes extend at right angles to the longitudinal axis of the plate (12).

7. Device according to Claim 1, characterized in that, on the side of the plate (12) facing away from the bone, the edge of the hole (10) is at least partially offset outwards by means of a bevelled surface (40) relative to the part of the wall forming the abutment (23).

8. Device according to Claim 1, characterized in that, on the side of the plate (12) adjacent to the bone, the walls of the hole (10) which are located on its longitudinal sides are also offset outwards relative to the clear hole width by means of a bevelled surface (46).

9. Device according to Claim 7 or 8, characterized in that, on the side of the plate (12) facing away from the bone, the edge of the hole (10) is offset outwards relative to the clear hole width by means of a bevel on the end face of the hole.

10. Device according to the preceding claims, characterized in that the length of the hole (10) corresponds to a shifting distance of at least one third of the shank diameter (d) of the screw inserted in it.

11. Device according to Claim 1, characterized in that, on at least some of the screws (16; 32-37), the screw shank has, between the screw head (17) and the threaded part (19), a threadless cylindrical intermediate piece (18), the diameter (d) of which is at least equal to the outside diameter of the threaded part (19).


**Revendications**

1. Dispositif pour stabiliser la zone d'une fracture osseuse ou d'une ostéotomie lors de l'ostéosynthèse par compression, consistant en une plaque (12) qui est destinée à être appliquée sur la région de l'os (14) à stabiliser et qui peut être assujettie aux parties de l'os au moyen de vis (16; 32 - 37) à têtes sphériques, la plaque (12) présentant au moins un trou (10) de réalisation oblongue dont les parois longitudinales forment, pour la tête (17) des vis, une contre-butée (23) qui est encaissée par rapport à la surface de la plaque et s'étend parallèlement à l'axe longitudinal (L) des trous,
caractérisé par le fait
que, du côté de la plaque (12) appliqué sur l'os, le bord du trou (10) est décalé vers l'extérieur par l'intermédiaire d'un biseau vis-à-vis de la largeur interne du trou, au moins à la face frontale de ce trou (10) proche de la fracture.

2. Dispositif selon la revendication 1, caractérisé par le fait que la contre-butée (23) formée par les parois longitudinales du trou (10) est définie, au moins en partie, par l'enveloppe d'un cylindre dont l'axe s'étend parallèlement à l'axe longitudinal (L) du trou.

3. Dispositif selon la revendication 1, caractérisé par le fait que la contre-butée (23) formée par les parois longitudinales du trou (10) est définie, au moins en partie, par deux plans qui se coupent sur une droite parallèle à l'axe longitudinal (L) du trou.

4. Dispositif selon la revendication 1, caractérisé par le fait que l'axe longitudinal (L) du trou (10) ou les axes longitudinaux d'au moins une partie des trous s'étendent parallèlement à l'axe longitudinal de la plaque (12).

5. Dispositif selon la revendication 1, caractérisé par le fait que l'axe longitudinal (L) du trou (10) ou les axes longitudinaux d'au moins une partie des trous s'étendent à l'oblique par rapport à l'axe longitudinal de la plaque (12).

6. Dispositif selon la revendication 1, caractérisé par le fait que l'axe longitudinal (L) du trou (10) ou les axes longitudinaux d'au moins une partie des trous s'étendent à angle droit par rapport à l'axe longitudinal de la plaque (12).

7. Dispositif selon la revendication 1, caractérisé par le fait que, au moins en partie du côté de la plaque (12) tourné à l'opposé de l'os, le bord du trou (10) est décalé vers l'extérieur par l'intermédiaire d'une surface biseautée (40), vis-à-vis de la partie de la paroi formant la contrebutée (23).

8. Dispositif selon la revendication 1, caractérisé par le fait que, du côté de la plaque (12) appliqué sur l'os, les parois longitudinales du trou (10) sont, elles aussi, décalées vers l'extérieur par l'intermédiaire d'une surface biseautée (46), vis-à-vis de la largeur interne du trou.

9. Dispositif selon la revendication 7 ou 8, caractérisé par le fait que, du côté de la plaque (12) tourné à l'opposé de l'os, le bord du trou (10) est décalé vers l'extérieur par l'intermédiaire d'un biseau, à la face frontale du trou, vis-à-vis de la largeur interne de ce trou.

10. Dispositif selon les revendications précédentes, caractérisé par le fait que la longueur du trou (10) correspond à une course de déplacement d'au moins un tiers du diamètre (d) de la tige de la vis qui y est engagée.

11. Dispositif selon la revendication 1, caractérisé par le fait que, pour au moins une partie des vis (16; 32-37), la tige de ces vis présente, entre la tête (17) de la vis et la région filetée (19), un tronçon intermédiaire cylindrique (18) exempt de filetage et dont le diamètre (d) est au moins égal au diamètre externe de la région filetée (19).

Fig. 0

Fig. 1

Fig. 2

0 053 999

Fig. 3

Fig. 4

Fig. 5

Fig. 6

Fig. 7

IX

0

59

62

R

67

12

IX

Fig. 8

65

66

β = 45°

E    S

VIII

r

59

r

62

Fig. 9

VIII

Fig. 10